# EUROPEAN PATENT APPLICATION

(11) **EP 4 299 578 A1**
(43) Date of publication of application: **03.01.2024**
(21) Application number: 22909618.5
(22) Date of filing: 21.11.2022
(51) Int. Cl.: C07H 1/06, C07H 3/02, C07C 29/76, C07C 31/24, C12P 7/18, C12N 1/16, C12R 1/645

(54) **METHOD FOR CO-PRODUCING ERYTHRITOL AND ARABINOSE FROM XYLOSE MOTHER LIQUOR**

(30) Priority: 26.12.2021 CN 202111606216
(71) Applicant: Zhejiang Huakang Pharmaceutical Co., Ltd., Quzhou, Zhejiang 324302 (CN)
(72) Inventor: WU, Aijuan, Quzhou, Zhejiang 324302 (CN); LUO, Jiaxing, Quzhou, Zhejiang 324302 (CN); HU, Changhui, Quzhou, Zhejiang 324302 (CN); YANG, Mingqian, Quzhou, Zhejiang 324302 (CN); LIAO, Chengjun, Quzhou, Zhejiang 324302 (CN); ZHENG, Yi, Quzhou, Zhejiang 342302 (CN); FANG, Shuncheng, Quzhou, Zhejiang 324302 (CN); LI, Mian, Santa Clara, California 95054 (US)
(74) Representative: Sun, Yiming
(86) International application number: PCT/CN2022/133112
(87) International publication number: WO 2023/116302

(57) **Abstract**

The present disclosure relates to a method of co-producing erythritol and arabinose by using xylose mother liquor, wherein an extract and a raffinate are obtained by separating the xylose mother liquor through a first chromatography, the extract is configured to prepare crystallized xylose, the raffinate and the liquid glucose or crystallized glucose are blended and erythritol is produced by using *Yarrowia lipolytica* with high osmotolerant and a high conversion rate. erythritol crystals are obtained by centrifugation and crystallization first by using characteristics of low solubility degree and easy crystallization of the erythritol, the arabinose raffinate having a high content of arabinose is obtained by separating a centrifuged erythritol mother liquor through a second chromatography, and arabinose crystals are obtained based on the arabinose raffinate. The present disclosure achieves an efficient utilization of the xylose mother liquor, and produces the erythritol of a higher added value by utilizing the glucose while obtaining the xylose and the arabinose, which reduces costs of fermentation and increases economic benefits.

## Description

### TECHNICAL FIELD

The present disclosure relates to the technical field of xylose mother liquor utilization, and in particular to methods of co-producing erythritol and arabinose by using xylose mother liquor.

### BACKGROUND

In a process of producing xylitol, a large amount of xylose mother liquor is produced. The xylose mother liquor has a high content of hetero sugars and is often sold as a by-product for making caramel coloring, etc., and the xylose mother liquor has a low added value. The xylose mother liquor contains xylose of 40%~60%, glucose of 10%~20%, arabinose of 15%~20%, mannose of 0-10% and galactose of 0-5%. Methods of processing and utilizing the xylose mother liquor are common, mainly extracting xylose and arabinose from the xylose mother liquor. In order to reduce the difficulty of extraction and separation, fermentation is carried out using bacteria or yeast, and the glucose or the galactose is consumed out as a carbon source for the growth of the bacteria, thereby improving an extraction efficiency of the xylose and the arabinose.

The patent publication No. CN112094956A uses saccharomyces cerevisiae to continuously ferment the xylose mother liquor, consume the glucose, and purify the xylose and the arabinose using chromatography. The patent No. CN101705253B uses a secondary fermentation to turn the xylose in mother liquor into xylitol and purify the arabinose at the same time. The patent No. CN102603814B similarly uses yeast fermentation to remove the glucose and the galactose from the xylose mother liquor diluted to 20% and obtains xylose and arabinose clear liquor. The patent No. CN101857523B removes the glucose and galactose from the mother liquor by fermenting, and a filtrated clear liquor is hydrogenated through decolorizing, ion changing and concentrating to prepare the xylitol and the arabinitol, and then two components are separated through chromatography, two finished products are obtained by crystallizing. The patent No. CN102952165B also removes the glucose and the galactose using fermentation, and then separates the crystals of the arabinose according to a difference of characteristics of the crystallization. The patent publication No. CN109504733A adopts *Pichia pastoris* and *Aureobasidium pullulans* to ferment for producing the erythritol, although the xylose mother liquor is used as carbon source, but a concentration of the erythritol is very low, only about 40g/L. Most of the above technologies of patents are related to removing the glucose or the galactose by strain fermentation and further separating the xylose and the arabinose to improve purity the xylose and the arabinose. But the above technologies all have some limitation, due to a high sugar level in the xylose mother liquor, bacteria are generally not suitable for growth. An osmotolerant yeast are mostly used in an aerobic fermentation, which needs for large compressed air, while an amount of diluted mother liquor is large. A utilization of all glucose in the mother liquor requires a larger amount of cells, and culture for multiple times. The fermentation time is 48h or longer, although a purpose of purification can be achieved, the consumption of energy, production cycle and cost are greatly increased.

### SUMMARY

A technical problem to be solved by the present disclosure is to provide a method for co-producing erythritol and arabinose by using a xylose mother liquor, to reduce a utilization cost of the xylose mother liquor, to increase a utilization value of glucose, meanwhile separating xylose and arabinose, producing an added-value product erythritol by using yeast fermentation, and increasing yield of the erythritol.

The present disclosure is achieved by providing the method for co-producing the erythritol and the arabinose by utilizing the xylose mother liquor, including:
Step 1: obtaining a xylose extract having a high content of xylose component and a xylose raffinate having a high content of glucose component respectively by separating the xylose mother liquor through a first simulated moving bed chromatography, and obtaining xylose crystals by concentrating and crystallizing the xylose extract.
Step 2: obtaining a concentrated xylose raffinate by concentrating the xylose raffinate and obtaining a glucose mixture by blending the concentrated xylose raffinate with liquid glucose or crystallized glucose, wherein the concentrated xylose raffinate has a solid content of 30%-50% and a glucose content of 9%~14%, the glucose mixture has a glucose content of 40% ~ 50%.
Step 3: obtaining a fermentation broth by inoculating a pre-prepared seed solution of a *Yarrowia lipolytica* into a fermentation medium of a fermenter, while adding a glucose mixture of step 2 for fermenting, wherein a glucose content of the fermentation broth is <0.3%; obtaining a fermentation filtrate by filtering the fermentation broth, and respectively obtaining erythritol crystals and a centrifuged erythritol mother liquor by decolorizing, ion exchanging, concentrating, centrifuging and crystallizing the fermentation filtrate in sequence.
Step 4: obtaining an erythritol extract having a high content of an erythritol component and an erythritol raffinate having a high content of an arabinose component respectively by separating the centrifuged erythritol mother liquor through a second simulated moving bed chromatography, and mixing the erythritol extract with the fermentation filtrate in step 3, and obtaining arabinose crystals by decolorizing, ion exchanging, concentrating, centrifuging and crystallizing the erythritol raffinate in sequence.

Relative to the prior art, in the method of the present disclosure for co-producing the erythritol and the arabinose, by utilizing the xylose mother liquor, the extract and the raffinate are obtained by separating the xylose using a first chromatography, the extract is configured to prepare crystallized xylose, the raffinate and the liquid glucose or crystallized glucose are blend to ferment and produce the erythritol by using a *Yarrowia lipolytica* of high osmotolerant with a high conversion rate, and the erythritol crystals are obtained by centrifuging and crystallizing the erythritol using characteristics of low solubility degree and easy crystallization of the erythritol, the raffinate having a high content of arabinose is obtained by separating an centrifuged erythritol mother liquor through a second chromatography, and the arabinose crystals are obtained by preparing. The present disclosure realizes an efficient utilization of the xylose mother liquor and obtains the xylose and the arabinose, while producing the erythritol of a higher added value by utilizing the glucose of the xylose and the arabinose. Meanwhile, by mixing the xylose with the liquid glucose or the crystallized glucose, on the one hand, the glucose content in the medium is increased, which improves the yield of the erythritol in each batch of fermentation, on the other hand, increasing a utilization rate of mother liquor in each batch of the fermentation, overall using the raffinate of the xylose mother liquor to prepare the erythritol by fermentation with fermentation broth, a concentration of the erythritol is above 156g/L, and a conversion rate is above 52%. The present disclosure also reduces costs of the fermentation, improves the added value of the xylose mother liquor, and increases economic benefits.

### BRIEF DESCRIPTION OF THE DRAWINGS

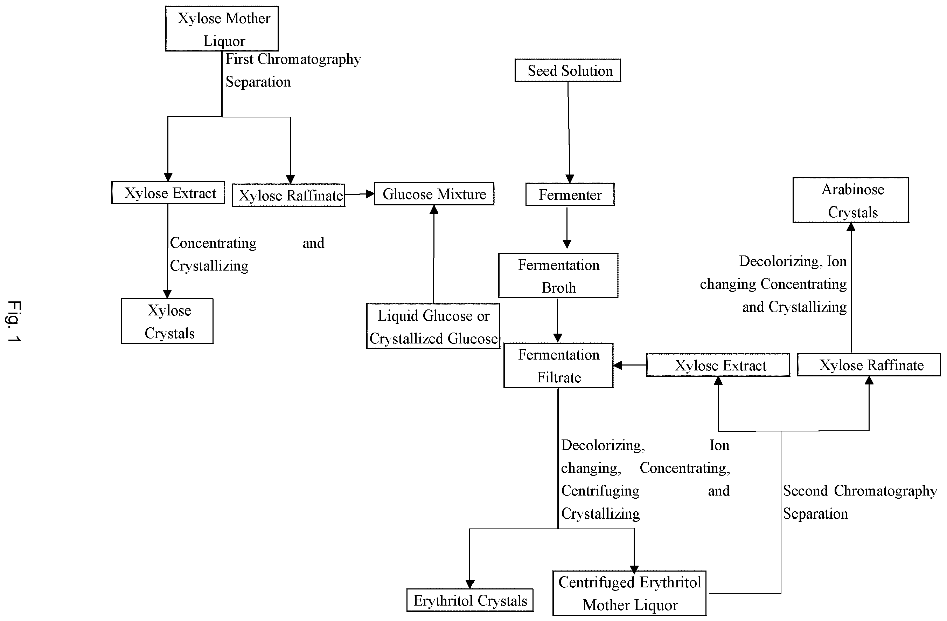

FIG. 1 illustrates a schematic diagram of a method for co-producing erythritol and arabinose by using a xylose mother liquor of the present disclosure.

### DETAILED DESCRIPTION

In order to make the technical problem to be solved, technical solution and beneficial effects of the present disclosure more clearly understood, the present disclosure is described in further detail hereinafter in conjunction with the accompanying drawings and embodiments. It should be understood that the specific embodiments described herein are used only to explain the present disclosure and are not intended to limit the present disclosure.

Referring to FIG 1, a preferred embodiment of the method for co-producing erythritol and arabinose by using xylose mother liquor includes the following operations:
Step 1: obtaining a xylose extract having a high content of xylose component and a xylose raffinate having a high content of glucose component respectively by separating a xylose mother liquor through a first simulated moving bed (SMB) chromatography, and obtaining xylose crystals by concentrating and crystallizing the xylose extract.
Step 2: obtaining a concentrated xylose raffinate by concentrating the xylose raffinate, and obtaining a glucose mixture by blending the concentrated xylose raffinate with liquid glucose or crystallized glucose, wherein the concentrated xylose raffinate has a solid content of 30%-50% (g/100mL) and a glucose content of 9%~14% (g/100mL), the glucose mixture has a glucose content of 40%~ 50%.
Step 3: obtaining a fermentation broth by inoculating a pre-prepared seed solution of *Yarrowia lipolytica* into a fermentation medium of a fermenter, while adding the glucose mixture of step 2 in the fermentation medium for fermentation, wherein a glucose content of the fermentation broth is <0.3%. A fermentation filtrate is obtained by filtering the fermentation broth, and respectively obtaining erythritol crystals and centrifuged erythritol mother liquor by decolorizing, ion exchanging, concentrating, centrifuging and crystallizing the fermentation filtrate in sequence.
Step 4: obtaining an erythritol extract having a high content of an erythritol component and an erythritol raffinate having a high content of an arabinose component respectively by separating the centrifuged erythritol mother liquor through a second simulated moving bed chromatography, and mixing the erythritol extract with the fermentation filtrate in step 3, and obtaining arabinose crystals by decolorizing, ion changing, concentrating and crystallizing the erythritol raffinate in sequence.

Specifically, the fermentation medium in step 3 is prepared by 25%-32% of glucose content, 0.5%~1% of a yeast paste, of corn pulp dry powder, 0.03%-0.08% of magnesium sulfate, 0.2%-0.8% of ammonium citrate and 0.02%-0.05% of dipotassium hydrogen phosphate.

Specifically, the pre-prepared seed solution of the *Yarrowia lipolytica* in step 3 is prepared according to the following operations: pre-preparing a slant test tube seed medium, inoculating a *Yarrowia lipolytica* strain into the slant test tube seed medium for cultivation to obtain a slant test tube seed culture, wherein the slant test tube seed medium is prepared by: 20%-25% of glucose, yeast paste of 0.8%-1.5% of yeast paste and 1.5%-2% of agar.

Specifically, the pre-prepared seed solution of the *Yarrowia lipolytica* in step 3 is prepared according to the following operations: pre-preparing a slant eggplant-type flask seed medium, inoculating a *Yarrowia lipolytica* strain into the slant eggplant-type flask seed medium for cultivation to obtain a slant eggplant-type flask seed culture, wherein the slant eggplant-type flask seed medium is prepared by: 20%-25% of glucose, 0.8%-1.5% of yeast paste and 1.5%-2% of agar.

Specifically, the pre-prepared seed solution of the *Yarrowia lipolytica* in step 3 is prepared according to the following operations: pre-preparing a shake flask seed medium, inoculating a *Yarrowia lipolytica* strain into a shake flask for cultivation to obtain a shake flask seed solution, wherein the shake flask seed medium is prepared by: 20%-25% of glucose, yeast paste of 0.8%-1.5%, 0.03%-0.08% of magnesium sulfate and 0.2%-0.7% of ammonium citrate.

Specifically, the pre-prepared seed solution of the *Yarrowia lipolytica* in step 3 is prepared according to the following operations: pre-preparing a fermenter seed medium, inoculating a *Yarrowia lipolytica* strain into the fermenter seed medium for cultivation to obtain the fermenter seed solution, wherein the fermenter seed medium is prepared by: 25%-30 30% of glucose, 0.5%-1.0% of yeast paste, 0.3%-0.8% of peptone, 0.03%-0.08% of magnesium sulfate and 0.2%-0.8% of ammonium citrate, an inoculation quantity is 5% W/V-10% W/V, an initial pH of fermentation is 6.0~7.0, a sterilization temperature of the fermenter seed medium is 115°C~121°C, sterilization time is 20min-30 min.

The method of the present disclosure is further described below in connection with specific examples.

### Example 1

A first example of the method for co-producing the erythritol and the arabinose by utilizing the xylose mother liquor includes the following operations.

The xylose mother liquor was separated by the first chromatography to obtain an extract containing xylose, and the extract was concentrated to have 80% of solids, and crystallized xylose was obtained by evaporating crystallization. The proportion of each component in a raffinate was 25%-32% of the glucose, 18%~25% of the arabinose, 5%-15% of the galactose. The raffinate was concentrated to have 40% of the solids, where the glucose content was 12%, and was mixed with the crystallized glucose to obtain a mixture, so that the glucose content of the mixture was 45%. A preparation system was 100L, and other additives of the fermentation medium other than the glucose were added proportionally to the fermentation medium for sterilization and set aside.

The fermentation system of 70L with 3 batches, the fermentation medium was fed during the fermentation process, the specific operations were as followed.

An initial fermentation volume was 40L, the medium was prepared according to the above formulation of the fermentation medium, an initial glucose content in the medium was 18%, which was prepared for sterilization, and set aside. Shake flasks of 500mL and 5L respectively with a filling liquid volume of 10% of the total volume were used to prepare shake flask seed cultures. The shake flask seeds were cultured for 20h~24h. When bacteria density (an absorbance value of bacterial solution in a wavelength of 600nm, also referred to as an OD value) in the shake flask of 5L was 18~25, the strain was inoculated into the fermenter, a volume of inoculum was 8%, a fermentation temperature was 30°C, a speed of rotation was 200rpm~400rpm, dissolved oxygen was 20%- 30%, a volume of aeration was 1.5Nm³/h. When a density of the bacteria in the fermentation broth reached 35~40, the above mixed sugar liquid was started to replenish to the fermenter to reach the volume to 70L. During a continuous feeding process, a glucose content in the fermentation broth was kept at 17%-20%. After the feeding was completed, the fermentation was continued, when the glucose content in the fermentation broth was <0.3%, the fermentation was stopped. Fermentation results were shown in Table 1.

**Table 1 Test results of example 1.**

| | Batch I | Batch II | Batch III |
|---|---|---|---|
| Time of Fermentation/h | 102 | 97 | 100 |
| Concentration of Erythritol /(g/L) | 156.74 | 165.40 | 162.80 |
| Conversion rate/% | 52.2 | 55.1 | 54.3 |

The concentration of erythritol were all more than 156 g/L, and the conversion rate was ≥52.2%.

When the fermentation was ended, the fermentation broth was filtered through ceramic membrane to obtain supernatant, which was decolorized, ion changed and concentrated to have solids of 68%. The solids then was cooled down and crystallized at a rate of 5°C/h, and after 20h, erythritol crystals were obtained by centrifugation. The erythritol component and the arabinose component were obtained by separating the centrifuged mother liquor through the second simulated moving bed chromatography, the erythritol component was returned to a filtration supernatant to increase production of the erythritol. A purity of the arabinose reached 75%, and the arabinose crystals were obtained by concentration, crystallization, and centrifugation.

### Example 2

A second example of the method for co-producing the erythritol and the arabinose by utilizing the xylose mother liquor includes the following operations:
The obtaining of the raffinate and the proportion of each component were the same as in Example 1. The raffinate was concentrated to have 50% of solids, where the glucose content was 14.3%, and liquid glucose was concentrated to have 65% of the glucose content, and the two were mixed to obtain the mixture in a ratio of 4:6 by volume, so that the glucose content of the mixture was 45%. A preparation system was 1000 L, and the other additives of the fermentation medium other than the glucose were added proportionally to the fermentation medium for sterilization and set aside.

The fermentation system of 70L with 3 batches, the fermentation medium was fed during the fermentation process, the specific operations were as followed:
An initial fermentation volume was 400L, the fermentation medium was prepared according to the above fermentation medium, an initial glucose content in the medium was 18.7%, which was prepared for sterilization and set aside. Slant test tube seeds were inoculated into slant eggplant-type flask of 500mL to prepare slant test tube seed cultures, and the slant test tube seeds were cultured in a temperature of 30°C for 4~5 days. 80mL of sterile water was added to each flask to wash down lawn. The lawn was respectively inoculated into the shake flasks of 5L for being cultured for 20h~24h in a temperature of 30°C. When bacteria density was 18~25, the strain was inoculated into two seed fermenters of 50L, where a filling liquid volume was 35L, a temperature of the fermentation was 30°C, a pressure of the fermenter was 0.1MPa. When bacteria density reached 20~25, the strain in one seed fermenter was inoculated into a fermenter of 1000 ml, where a rotation speed was 180rpm ~ 300rpm, dissolved oxygen was 20% ~ 30%, an aeration volume was 16Nm³ / h. When bacteria density reached 35 ~ 40, the above mixed sugar solution of 300L was start to replenish to the seed fermenter, and meanwhile a new seed solution from another seed fermenter was added to the seed fermenter. During a continuous feeding process, a glucose content in the fermentation broth was kept at 17%-20%. After the feeding was completed, the fermentation was continued, when the glucose content in the fermentation broth was<0.3%, the fermentation was stopped, a total concentration of the glucose of the fermentation system was 30%. The fermentation results were as shown in the following Table.

| | Batch I | Batch II | Batch III |
|---|---|---|---|
| Time of Fermentation/h | 104.5 | 101 | 106.5 |
| Concentration of Erythritol/(g/L) | 160.71 | 165.40 | 162.80 |
| Conversion rate/% | 53.9 | 55.5 | 54.6 |

The concentration of erythritol were all more than 160 g/L, and the conversion rate was ≥53.9%.

When the fermentation was ended, the fermentation broth was filtered through the ceramic membrane to obtain the supernatant, which was decolorized, ion changed and concentrated to have solids of 65%. The solids then was cooled down and crystallized at a rate of 6°C/h, and after 20h, the erythritol crystals were obtained by centrifugation. The erythritol component and the arabinose component were obtained by separating the centrifuged mother liquor through the second simulated moving bed chromatography, the erythritol component was returned to the filtration supernatant. A purity of the arabinose reached 75%, and the arabinose crystals were obtained by concentration, crystallization, and centrifugation.

### Example 3

A third example of the method for co-producing the erythritol and the arabinose by utilizing the xylose mother liquor, including the following operations:
The obtaining of the raffinate and the proportion of each component were the same as in the Example 1 and the Example 2. The raffinate was concentrated to have 48% of solids, where the glucose content was 13.7%. 550L of water and 380kg of crystalline dextrose monohydrate and tap water then were added to the solids to obtain the mixture, the glucose content of the mixture was 42%. The preparation system was 1000L, and the other additives of the fermentation medium other than the glucose were added proportionally to the fermentation medium for sterilization and set aside.

The fermentation system of 700L with 3 batches, the fermentation medium was fed during the fermentation process, the specific operations were as followed:
An initial fermentation volume of 400L, the medium was prepared according to the above fermentation of the medium formulation, an initial glucose content in the medium was 20%, which was prepared for sterilization and set aside. The slant test tube seeds were inoculated into slant eggplant-type flasks of 500mL to prepare the slant test tube seed cultures, and the slant test tube seeds were cultured in the temperature of 30°C for 4~5 days. 80mL of sterile water was added to each flask to wash down the lawn. The lawn was respectively inoculated into the shake flask of 5L for being cultured for 22h in the temperature of 30°C. When bacteria density was 18~25, the strain was inoculated into the two seed fermenters of 50L, where the filling liquid volume was 35L, the temperature of the fermentation was 30°C, the pressure of the fermenter was 0.1MPa. When the bacteria density reached 20~25, the strain of one seed fermenter of two seed fermenters was inoculated into the fermenter of 1000ml, where a rotation speed was 180rpm ~ 300rpm, the dissolved oxygen was 20% ~ 30%, an aeration volume was 15Nm³ / h. When the bacteria density reached 35 ~ 40, the above mixed sugar solution of 300L was start to replenish to the seed fermenter, and meanwhile a new seed solution from another seed fermenter was added to the seed fermenter. During a continuous feeding process, the glucose content in the fermentation broth was kept at 15%-18%. After the feeding was completed, the fermentation was continued, when the glucose content in the fermentation broth was <0.3%, the fermentation was stopped, a total concentration of the glucose of the fermentation system was 30%. The fermentation results were as shown in the following Table.

| | Batch I | Batch II | Batch III |
|---|---|---|---|
| Time of Fermentation/h | 99.5 | 104 | 102.5 |
| Concentration of Erythritol/(g/L) | 163.57 | 159.80 | 165.22 |
| Conversion rate/% | 54.5 | 53.3 | 55.1 |

The concentration of erythritol were all more than 159 g/L, and the conversion rate was ≥53.3%.

When the fermentation was ended, the fermentation broth was filtered through the ceramic membrane to obtain the supernatant, which was decolorized, ion changed and concentrated to have solids of 67%. The solids then was cooled down and crystallized at a rate of 6°C/h, and after 18h, the erythritol crystals were obtained by centrifugation. The erythritol component and the arabinose component were obtained by separating the centrifuged mother liquor through the second simulated moving bed chromatography, the erythritol component was returned to the filtration supernatant. A purity of the arabinose reached 78%, and the arabinose crystals were obtained by concentration, crystallization, and centrifugation.

### Comparative example 1

The erythritol was prepared by direct fermentation using chromatography raffinate having solids of 30%, an initial glucose content was 7.2%. Except for the feeding fermentation, a 70L fermentation system and a fermentation control of Example 1 were used. When the fermentation was ended, the concentration of the erythritol was 31.6 g/L, and a conversion rate was 43.8%.

### Comparative example 2

After the chromatography raffinate having solids of 30-40% are mixed with the crystallized glucose, the liquid glucose and the dextrose monohydrate, all of them were configured to be used as the initial fermentation medium. An initial glucose content was 21%. Except for the feeding fermentation process, the70L fermentation system and the fermentation control of Example 1are used. When the fermentation is ended, the concentration of the erythritol was 64.5g/L, and a conversion rate was 30.0%.

The above are merely preferred embodiments of the present disclosure, and is not intended to limit the invention. Any amendments, equivalent replacement and corrections made within the spirit and principles of the present disclosure should be included in the scope of protection of the present disclosure.

## Claims

1. A method for co-producing erythritol and arabinose by using a xylose mother liquor, comprising:
Step 1: obtaining a xylose extract having a high content of xylose component and a xylose raffinate having a high content of glucose component respectively by separating the xylose mother liquor through a first simulated moving bed chromatography, and obtaining xylose crystals by concentrating and crystallizing the xylose extract;
Step 2: obtaining a concentrated xylose raffinate by concentrating the xylose raffinate, and obtaining a glucose mixture by blending the concentrated xylose raffinate with liquid glucose or crystallized glucose, wherein the concentrated xylose raffinate has a solid content of 30%-50% and a glucose content of 9%-14%, the glucose mixture has a glucose content of 40%-50%;
Step 3: obtaining a fermentation broth by inoculating a pre-prepared seed solution of *Yarrowia lipolytica* into a fermentation medium of a fermenter, while adding a glucose mixture of step 2 for fermenting, wherein a glucose content of the fermentation broth is <0.3%; obtaining a fermentation filtrate by filtering the fermentation broth, and respectively obtaining erythritol crystals and a centrifuged erythritol mother liquor by decolorizing, ion changing, concentrating, centrifuging and crystallizing the fermentation filtrate in sequence; and
Step 4: obtaining an erythritol extract having a high content of an erythritol component and an erythritol raffinate having a high content of an arabinose component respectively by separating the centrifuged erythritol mother liquor through a second simulated moving bed chromatography, and mixing the erythritol extract with the fermentation filtrate in step 3, and obtaining arabinose crystals by decolorizing, ion changing, concentrating and crystallizing the erythritol raffinate in sequence.

2. The method for co-producing the erythritol and the arabinose by utilizing the xylose mother liquor of claim 1, wherein the fermentation medium in the step 3 is prepared by 25%-32% of glucose, 0.5%-1% of yeast extract, 0.3%-0.8% of corn pulp dry powder, 0.03%-0.08% of magnesium sulfate, 0.2%-0.8% of ammonium citrate, and 0.02%-0.05% of dipotassium hydrogen phosphate.

3. The method for co-producing the erythritol and the arabinose by utilizing the xylose mother liquor of claim 1, wherein in step 3, the pre-prepared seed solution of the *Yarrowia lipolytica* is prepared according to the following operations: pre-preparing a slant test tube seed medium, inoculating a *Yarrowia lipolytica* strain into the slant test tube seed medium for cultivation to obtain a slant test tube seed culture, wherein the slant test tube seed medium is prepared by: 20%-25% of glucose, 0.8%-1.5% of yeast paste and 1.5%-2% of agar.

4. The method for co-producing the erythritol and the arabinose by utilizing the xylose mother liquor of claim 1, wherein in step 3, the pre-prepared seed solution of the *Yarrowia lipolytica* is prepared according to the following operations: pre-preparing a slant eggplant-type flask seed medium, inoculating a *Yarrowia lipolytica* strain into the slant eggplant-type flask seed medium for cultivation to obtain an slant eggplant-type flask seed culture, wherein the slant eggplant-type flask seed medium is prepared by: 20%-25% of glucose, 0.8%-1.5% of yeast paste and 1.5%-2% of agar.

5. The method for co-producing the erythritol and the arabinose by utilizing the xylose mother liquor of claim 1, wherein in step 3, the pre-prepared seed solution of the *Yarrowia lipolytica* is prepared according to the following operations: pre-preparing a shake flask seed medium, inoculating a *Yarrowia lipolytica* strain into a shake flask for cultivation to obtain a shake flask seed solution, wherein the shake flask seed medium is prepared by: 20%-25% of glucose, 0.8%-1.5% of yeast paste, 0.03%-0.08% of magnesium sulfate and 0.2%-0.7% of ammonium citrate.

6. The method for co-producing the erythritol and the arabinose by utilizing the xylose mother liquor of claim 1, wherein, in step 3, the pre-prepared seed solution of the *Yarrowia lipolytica* is prepared according to the following operations: pre-preparing a fermenter seed medium, inoculating a *Yarrowia lipolytica* strain into the fermenter seed medium for cultivation to obtain a fermenter seed solution, wherein the fermenter seed medium is prepared by: 25%-30% of glucose, 0.5%-1.0% of yeast paste, 0.3%-0.8% of peptone, 0.03%-0.08% of magnesium sulfate and 0.2%-0.8% of ammonium citrate, an inoculation quantity is 5% W/V-10% W/V, an initial pH of fermentation is 6.0-7.0, a sterilization temperature of the fermenter seed medium is 115°C-121°C, sterilization time is 20min-30 min.
